# EUROPEAN PATENT SPECIFICATION

(11) **EP 1 750 677 B1**
(45) Date of publication and mention of the grant of the patent: **01.02.2017**
(21) Application number: 05742209.9
(22) Date of filing: 27.05.2005
(51) Int. Cl.: A61K 9/20, A61K 9/46, A61K 9/00

(54) **ORAL THERAPEUTIC COMPOUND DELIVERY SYSTEM**
ABGABESYSTEM FÜR EINE ORALE THERAPEUTISCHE VERBINDUNG
SYSTEME D'ADMINISTRATION ORALE DE COMPOSE THERAPEUTIQUE

(30) Priority: 28.05.2004 US 575461 P
(43) Date of publication of application: 14.02.2007
(73) Proprietor: IMAGINOT PTY LTD., Coorparoo DC, QLD 4151 (AU); Johnson & Johnson Consumer Inc., Skillman, NJ 08558 (US)
(72) Inventor: ROBERTS, Michael, Stephen, West Lake, Queensland 4074 (AU); SARKAR, Mantu, Fairfield, QLD 4103 (AU); DAVIDSON, George Alexander, larnook, NSW 2480 (AU); JIANG, Ruoying, Sherwood, QLD 4075 (AU); ELLIOTT, Geraldine, Ann, Mount Ommaney, QLD 4074 (AU); BEZANEHTAK, Kelvan, Rosebery, NSW 2018 (AU); CHANDLER, Stephen Douglas, Mayfield, NSW 2304 (AU); DAVEY, Greg, Sinnamon Park, QLD 4073 (AU)
(74) Representative: Delorme, Nicolas
(86) International application number: PCT/AU2005/000759
(87) International publication number: WO 2005/115345

(56) References cited:
- EP-A1- 0 505 872
- WO-A1-02/100391
- WO-A1-2004/017976
- WO-A1-2007/059591
- WO-A2-98/38983
- CA-A1- 2 326 809
- DE-A1- 19 814 392

## Description

### BACKGROUND OF THE INVENTION

### FIELD OF THE INVENTION

The present invention relates generally to therapeutic formulations. More particularly, this present invention provides an oral delivery system for a therapeutic compound that is a base, a salt of a base, an amphoteric compound or a salt of an amphoteric compound with pharmacological, physiological or biochemical activity or a proactive form thereof. The present invention even more particularly provides a swallow formulation comprising a therapeutic compound that is a base, a salt of a base, an amphoteric compound or a salt of an amphoteric compound which facilitates the rapid delivery of the therapeutic compound to the circulatory system.

### DESCRIPTION OF THE PRIOR ART

Bibliographic details of the publications referred to in this specification are also collected at the end of the description.

Reference to any prior art in this specification is not, and should not be taken as, an acknowledgment or any form of suggestion that this prior art forms part of the common general knowledge in any country.

Improving the rate and extent of absorption of oral formulations of compounds has been the subject of substantial research. In general, once a solid swallow composition reaches the stomach, it undergoes disintegration and/or dissolution and passes into the small intestine where the active ingredient is absorbed across intestinal walls into the circulatory system *via* the portal vein and liver before reaching the site of action.

Effervescent tablet formulations which are disintegrated and/or dissolved in water prior to administration are well known. Such formulations generally contain effervescent couples such as citric acid and sodium bicarbonate in large amounts. For example, US 6,245,353 describes a tablet containing cetirizine and an effervescent couple for disintegration in water prior to administration. A variety of effervescent formulations which are intended to be dispersed and/or dissolved prior to administration are disclosed for example in US Patent No. 4,704,269, US Patent No. 4,309,408 and US Patent No. 4,942,039.

Some publications teach the inclusion of about 630 mg sodium bicarbonate in swallow tablets so as to provide isotonic conditions in the stomach. US Patent No. 6,316,025, for example, describes a swallow tablet of paracetamol containing 300 mg to 1000 mg of sodium bicarbonate per tablet and a paracetamol to sodium bicarbonate ratio of between 0.74 and 1. Grattan et al., Eur. J. Pharm. Biopharm 49(3):225-229, 2000, subsequently reported that a formulation with 630 mg sodium bicarbonate provided improved pharmacokinetic outcomes. It was suggested that this was due to an osmotic effect of sodium bicarbonate, which would be isotonic when ingested with 100 mL of water. US Patent Application No. 20040204475 describes a formulation containing sodium bicarbonate and eletriptan. The sodium bicarbonate is administered in an amount to obtain a duodenal concentration approximately isotonic with serum (150 millimoles). The formulations exemplified all contained 630 mg sodium bicarbonate.

US Patent Application No. 20050032867 describes a fast disintegrating and dispersing sumatriptan formulation comprising about 5 to about 50% by weight base component. The base component of the formulation reacts with the acid component of the stomach, sumatriptan or acid component of the tablet to generate gas so as to facilitate the disintegration and dispersion of the tablet.

It is widely accepted that raising the pH will inhibit the dissolution of basic compounds. WO 2004/017976 describes a fast dissolving and taste masked oral dosage form comprising the basic compound sildenafil. The specification describes the use of any pharmaceutically acceptable pH raising agent to inhibit dissolution of sildenafil, preventing dissolution of sildenafil in the mouth and thus masking the taste of the sildenafil. Agents that raise or increase the pH include sodium carbonate, sodium bicarbonate, calcium carbonate and magnesium carbonate.

Furthermore, precipitation of the basic compound ondansetron in alkaline solutions containing sodium bicarbonate has been reported (Jaronsinski P F and Hirschfeld S, N. Eng. J. Med. 325:1315-1316, 1991).

A relatively large amount of prior art deals with the use of sodium bicarbonate and other pH modulating agents to affect the absorption of acidic drugs, particularly acidic Non-steroidal Anti-Inflammatory Drugs (NSAIDs) and their salts. WO9744023 deals with the use of sodium and potassium bicarbonate to enhance absorption of salts of diclofenac. US patent 4,834,966 and others deal with the use of arginine, ibuprofen and sodium bicarbonate formulations to enhance absorption of ibuprofen. US patent 4704405 deals with the use of sodium sulindac, a base and a bicarbonate to improve absorption of sulindac. The enhanced absorption results from the increased solubility of acidic drugs at elevated pH, owing to greater ionisation of acidic groups. Neuvonen, P. J. and Kivisto, K. T. (Clin. Pharmacokinet. 27 (2) 120-8, 1994.) state that several drugs show enhanced absorption in the presence of pH modulating agents such as common antacids of sodium bicarbonate and magnesium hydroxide due to these antacids increasing gastric pH and thus increasing solubility.

WO02100391, WO9838983, EP0505872 disclose swallow formulations containing paracetamol, in the case of EP0505872 pseudoephedrine. For WO9838983 and EP0505872 the content of sodium bicarbonate is above 200 mg per tablet.

On the basis of these disclosures, it would be expected that the addition of bases such as carbonates to therapeutic compounds that are bases, salts of bases, amphoteric compounds or salts of amphoteric compounds, will reduce their solubility and hence dissolution as a result of the increased pH. Unexpectedly, we have found that in the case of basic drugs, where increased pH is likely to lead to lower solubility and hence worse dissolution and absorption, the use of pH modulating agents can still achieve increased dissolution and potentially increased absorption.

In accordance with the present invention, therapeutic compositions are defined in which the addition of bases such as carbonates, to therapeutic compounds that are bases, salts of bases, amphoteric compounds or salts of amphoteric compounds, enable *enhanced in vitro* dissolution of the therapeutic agent.

### SUMMARY OF THE INVENTION

The present invention relates generally to therapeutic formulations and more particularly fast dissolving swallow formulations for a therapeutic compound that is a base, a salt of a base, an amphoteric compound or a salt of an amphoteric compound with pharmacological, physiological or biochemical activity or a proactive form thereof. In particular, the present invention provides a swallow formulation comprising a therapeutic compound that is a base, a salt of a base, an amphoteric compound or a salt of an amphoteric compound with an appropriate amount of one or more pH modulating agents wherein at least one pH modulating agent is a carbonate. Preferably, the swallow formulation further comprises an agent which facilitates water uptake. The swallow formulation of the present invention exhibits enhanced dissolution of the therapeutic compound from the formulation.

In essence, the swallow formulation comprises a therapeutic compound that is a base, a salt of a base, an amphoteric compound or a salt of an amphoteric compound and one or more pH modulating agents wherein at least one pH modulating agent is a sodium or potassium bicarbonate in an amount that will neutralise 0.01 to 9.0 millimoles of hydrochloric acid and is present in an amount from about 1% to about 50% by weight of the swallow formulation, the amount of sodium or potassium bicarbonate being from 1 to 199 mg per tablet, the water uptake agent, wherein at least about 70% of the therapeutic compound is dissolved from the swallow formulation within 180 seconds in United States Pharmacopoeia (USP) dissolution apparatus 2 with 900 mL 0.0033 N hydrochloric acid at 30 rpm and 37°C. This quantity of dissolution medium contains 3 millimoles of hydrochloric acid. In addition, the fast dissolving oral delivery system may contain a combination of pharmaceutically acceptable excipients or other components such as disintegrants, preservatives, colors, antioxidants, emulsifiers, sweeteners, flavoring agents, binders, glidants and lubricants. In an exemplary form, the fast dissolving delivery system may also contain one or more pharmaceutically active agents. The oral solid dosage form may be administered by swallowing with water or any other liquid.

Particularly useful active agents include analgesics, anti-allergenics, anti-nausea agents, anti-migraine agents, agents for treating erectile dysfunction and hypnotics.

An appropriate amount of pH modulating agent is an amount sufficient to enhance the dissolution of the therapeutic compound from the swallow formulation. This amount will vary depending on the therapeutic compound. Preferably the pH modulating agent will be in an amount so as not to increase the pH of a 900 mL 0.0033 N hydrochloric acid dissolution medium that contains 3 millimoles of hydrochloric acid beyond 6.

Another aspect of the invention provides a dosage form such as a coated tablet, uncoated tablet, capsule, powder, paste, cachet, colloid, gel or melt.

The present invention further contemplates a method for delivering a therapeutic compound that is a base, a salt of a base, an amphoteric compound or a salt of an amphoteric compound by oral delivery including administration such as by swallowing, the method comprising orally delivering, including administering, a formulation comprising a therapeutic compound with an appropriate amount of one or more pH modulating agents wherein at least one of the pH modulating agents is a carbonate so as to enhance the dissolution of the therapeutic compound from the swallow formulation.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

### DETAILED DESCRIPTION OF THE PREFERRED EMBODIMENTS

The present invention provides a fast dissolving oral dosage form including a swallow formulation for a therapeutic compound that is a base, a salt of a base or an amphoteric compound or a salt of an amphoteric compound with pharmacological, physiological or biochemical activity or a proactive form thereof, and in particular swallow formulations. The oral dosage form generally comprises a therapeutic compound that is a base, a salt of a base, an amphoteric compound or a salt of an amphoteric agent combined with one or more pH modulating agents wherein at least one of the pH modulating agents is a carbonate. The oral dosage form of the present invention may also comprise a water uptake agent and may optionally be administered with water. Reference hereinafter to a "therapeutic compound" includes any pharmacologically, physiologically or biochemically active compound or proactive form thereof.

Accordingly, the present invention provides a swallow formulation comprising a therapeutic compound that is a base, a salt of a base, an amphoteric compound or a salt of an amphoteric compound and one or more pH modulating agents wherein at least one pH modulating agent is a sodium or potassium bicarbonate in an amount that will neutralise 0.01 to 9.0 millimoles of hydrochloric acid and is present in an amount from about 1% to about 50% by weight of the swallow formulation the amount of sodium or potassium bicarbonate being from 1 to 199 mg per tablet and a water uptake agent, and wherein at least about 70% of the therapeutic compound is dissolved from the swallow formulation within 180 seconds in USP dissolution apparatus 2 with 900 mL 0.0033 N hydrochloric acid at 30 rpm and 37°C.

It is to be understood that unless otherwise indicated, the subject invention is not limited to specific formulation components, manufacturing methods, dosage regimens, or the like, as such may vary. It is also to be understood that the terminology used herein is for the purpose of describing particular embodiments only and is not intended to be limiting.

It must also be noted that, as used in the subject specification, the singular forms "a", "an" and "the" include plural aspects unless the context clearly dictates otherwise. Thus, for example, reference to "therapeutic compound" includes a single therapeutic compound, as well as two or more therapeutic compounds; reference to "a pH modulating agent" includes a single pH modulating agent, as well as two or more pH modulating agents; reference to "a water uptake agent" includes a single water uptake agent, as well as two or more water uptake agents; and so forth.

In describing and claiming the present invention, the following terminology is used in accordance with the definitions set forth below.

A "swallow formulation" is any formulation which is administered to a subject by the action of swallowing the dosage form intact. The dosage form comprising the swallow formulation may be a coated tablet or capsule which does not have the same dissolution characteristics of the swallow formulation contained therein.

The terms "therapeutic compound", "compound", "pharmacologically active agent", "medicament", "active", "active ingredient", "drug" and "drug component" are used interchangeably throughout this specification. The terms also encompass pharmaceutically acceptable and pharmacologically active ingredients of those active agents specifically mentioned herein including but not limited to salts, esters, amides, pro-drugs, active metabolites, analogs and the like. When the terms "active agent", "compound", "pharmacologically active agent", "medicament", "active", "drug", and "drug component" are used, then it is to be understood that this includes those compounds *per se* as well as pharmaceutically acceptable, pharmacologically active salts, esters, amides, pro-drugs, metabolites, analogs, etc. The terms "agent", "compound" etc may be a single molecule or a composite of molecules.

By the term "effective amount" or "therapeutically effective amount" of a therapeutic compound as used herein means that a sufficient amount of a therapeutic compound is used to provide the desired therapeutic effect or the desired physiological or biochemical event including the amelioration of symptoms being treated or prevented. Of course, undesirable effects, e.g. side effects, are sometimes manifested along with the desired therapeutic effect; hence, a practitioner balances the potential benefits against the potential risks in determining what is an appropriate "effective amount".

The terms "delivery" and "administration" are used interchangeably throughout the specification to mean the act of providing the oral dosage form to an individual. The term "administering" is considered herein synonymous with "delivering", "providing", "introducing" or "swallowing".

By "pharmaceutically acceptable excipient" is meant a pharmaceutical vehicle comprised of a material that is not biologically or otherwise undesirable, i.e. the oral dosage form may be administered to a subject along with a therapeutic compound without causing any or a substantial adverse reaction. Excipients may include carriers and other additives such as diluents, binders, detergents, coloring agents, flavoring agents, wetting or emulsifying agents, preservatives, glidants, lubricants and the like as well as disintegrants.

The terms "treating" and "treatment" as used herein refer to reduction or amelioration in severity and/or frequency of symptoms, elimination of symptoms and/or underlying cause and/or prevention of the occurrence of symptoms and/or their underlying cause. Thus, for example, "treating" a patient involves prevention of a particular disorder or adverse physiological event in a susceptible individual as well as treatment of a clinically symptomatic individual by inhibiting or causing regression of a particular condition. Thus, for example, a method of treating a patient in need of pain relief encompasses both prevention of pain as well as treating conditions of pain.

"Patient" as used herein refers to an animal, preferably a mammal and more preferably human who can benefit from the pharmaceutical formulations and methods of the present invention. There is no limitation on the type of animal that could benefit from the presently described pharmaceutical formulations and methods. A patient regardless of whether a human or non-human animal may be referred to as an individual, subject, animal, host or recipient. The compounds and methods of the present invention have applications in human medicine, veterinary medicine as well as in general, domestic or wild animal husbandry. For convenience, an "animal" includes an avian species such as a poultry bird, an aviary bird or game bird.

The preferred animals are humans or other primates, livestock animals, laboratory test animals, companion animals or captive wild animals. A human is the most preferred target.

A "pH modulating agent" includes one or more than one pH modulating agents which alter the pH of an aqueous solution. These may include acids, bases or a combination of one or more acids and/or bases.

The carbonate may be any pharmaceutically acceptable carbonate or a mixture thereof. Reference to a "carbonate" includes a single agent or multiple (i.e. two or more) agents. Preferred carbonates include but are not limited to sodium carbonate, sodium bicarbonate, calcium carbonate, magnesium carbonate, ammonium carbonate, ammonium bicarbonate, potassium bicarbonate, sodium glycine carbonate, disodium glycine carbonate, arginine carbonate, lysine carbonate and/or other pharmaceutically acceptable carbonates or homologs or functional equivalents thereof and combinations thereof.

Other pH modulating agents may be pharmaceutically acceptable acids or acidic salts including citric acid, tartaric acid, succinic acid, ascorbic acid, malic acid, fumaric acid, metatartaric acid, adipic acid, sodium acid citrate, potassium acid citrate, glycine citrate, potassium acid tartrate, sodium acid tartrate, aspartic acid, glutamic acid, glycine, leucine, tyrosine, tryptophan, glycine fumarate, glycine hydrochloride, monophosphate glycine and combinations thereof.

A "water uptake agent" is any agent which will facilitate the uptake of water by absorbing, dissolving in or wicking water, used alone or in combination.. These may include wicking agents, disintegrants, binders, carriers and other hydrophilic excipients. Generally, but not exclusively, a "water uptake agent" facilitates uptake of water into the swallow formulation.

Accordingly, one aspect of the present invention provides a fast dissolving oral dosage form for a therapeutic compound and in particular a swallow formulation comprising a therapeutic compound. The oral dosage form generally comprises a therapeutic compound that is a base, a salt of a base, an amphoteric compound or a salt of an amphoteric compound and one or more pH modulating agents wherein at least one pH modulating agent is a carbonate in an amount that will neutralise 0.01 to 9.0 millimoles of hydrochloric acid and is present in an amount from about 1% to about 50% by weight of the swallow formulation and wherein at least 70% of the therapeutic compound is dissolved from the swallow formulation within 180 seconds in USP dissolution apparatus 2 with 900 mL 0.0033 N hydrochloric acid at 30 rpm and 37°C.

Examples of suitable active agents include analgesics e.g. opiates and opiate analogs, antipyretics, anti-migraine agents, sedatives, hypnotics, anti-anxiety agents, antipsychotic agents, antidepressants, anticonvulsants, antiemetics, antinauseants, expectorants, antitussives and decongestants, bronchodilators, antihistamines and anti-allergy agents, anti-diarrhoeals, antispasmodics and motility agents, hyperacidity, reflux and ulcer agents, antibiotics, antivirals and antifungals, detoxifying agents and agents used in drug dependence / withdrawal and erectile dysfunction agents.

Preferred therapeutic compounds are those which have one or more base groups such as but not limited to rizatriptan, zolmitriptan, zolpidem, zopiclone, fexofenadine, loratadine, ondansetron, tadalafil, vardenafil, sildenafil, ranitidine, famotidine, celecoxib, codeine, fentanyl, tramadol, pseudoephedrine, phenylpropanolamine, dextromethorphan, chlorpheniramine, diphenhydramine, cetirizine, and cimetidine and pharmaceutically acceptable salts thereof.

Preferably the carbonate is present in an amount from about 1% to about 45% by weight of swallow formulation and in an amount that will neutralise between 0.01 and 9.0 millimoles of hydrochloric acid. More preferably the carbonate is present in an amount from about 1% to about 40% by weight in the swallow formulation and in an amount that will neutralise between 0.02 and 8.0 millimoles of hydrochloric acid.

Examples of particular amounts of carbonate include 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50% by weight of swallow formulation.

Conveniently the carbonate component in the pH modulating agent is present in an amount from about 1 mg to about 450 mg in the swallow formulation.

Examples of particular amounts of carbonate include 1, 2, 3, 4, 5, 6, 7, 8, 9, 10,11, 12, 13, 14, 15, 16, 17, 18, 19, 20, 21, 22, 23, 24, 25, 26, 27,.28, 29, 30, 31, 32, 33, 34, 3, 36, 37, 3, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449 or 450 mg per swallow formulation.

More preferably the carbonate is present in an amount from about 2 mg to 400 mg.

Preferably at least one of the carbonates is soluble and/or dispersible.

Examples of suitable carbonates include, without being limited to sodium carbonate, sodium bicarbonate, calcium carbonate, magnesium carbonate, ammonium carbonate, ammonium bicarbonate, potassium bicarbonate, sodium glycine carbonate, disodium glycine carbonate, arginine carbonate, lysine carbonate and/or other pharmaceutically acceptable carbonates or homologs or functional equivalents thereof and combinations thereof.

Preferably, the carbonates of the swallow formulation are soluble and/or dispersible carbonates such as sodium bicarbonate or potassium bicarbonate or magnesium carbonate or combinations thereof.

Optionally the swallow formulation may contain further pH modulating agents such as pharmaceutically acceptable acids or acidic salts including citric acid, tartaric acid, succinic acid, ascorbic acid, malic acid, fumaric acid, metatartaric acid, adipic acid, sodium acid citrate, potassium acid citrate, glycine citrate, potassium acid tartrate, sodium acid tartrate, aspartic acid, glutamic acid, glycine, leucine, tyrosine, tryptophan, glycine fumarate, glycine hydrochloride, monophosphate glycine and combinations thereof.

In one swallow formulation embodiment the carbonate is sodium bicarbonate and/or potassium bicarbonate and/or magnesium carbonate and is present in an amount from about 1% to 50% by weight of the swallow formulation.

Suitable water uptake agents include cross-linked polyvinylpyrrolidone (crospovidone), croscarmellose sodium, sodium starch glycolate, starch, starch derivatives, hydroxypropylcellulose, low substituted hydroxypropylcellulose, hydroxypropylmethylcellulose, alginic acid, sodium alginate, calcium sulfate, calcium carboxymethylcellulose, microcrystalline cellulose, powdered cellulose, colloidal silicon dioxide, docusate sodium, guar gum, magnesium aluminium silicate, methylcellulose, polacrilin potassium, silicified microcrystalline cellulose, magnesium oxide, tragacanth, mannitol, sorbitol, xylitol, sucrose, lactose, fructose, maltose, polyethylene glycol, aminoacids, cyclodextrin, urea and/or polyvinylpyrrolidone (povidone, PVP).

The water uptake agent may be present in an amount from 5% to 95% by weight of the swallow formulation and more preferably between 10% and 90% by weight of the swallow formulation.

Preferably, the ratio of water uptake agent to pH modulating agent is between 0.1:1 and 20:1 by weight such as 0.1:1, 0.2:1, 0.3:1, 0.4:1, 0.5:1, 0.6:1, 0.7:1, 0.8:1, 0.8:1, 0.9:1, 1:1, 2:1, 3:1, 4:1, 5:1, 6:1, 7:1, 8:1, 9:1, 1.0:1, 11:1, 12:1, 13:1, 14:1, 15:1, 16:1, 17:1, 18:1, 19:1 or 20:1. More preferably the ratio of water uptake agent to pH modulating agent is between 0.3:1 and 15:1 by weight.

In one embodiment at least 80% of the therapeutic compound is dissolved from the swallow formulation within 180 seconds in USP dissolution apparatus 2 with 900 mL 0.0033 N hydrochloric acid at 30 rpm and 37°C. Even more preferably, at least 90% is dissolved in 180 seconds.

In another embodiment at least 70% of the therapeutic compound is dissolved from the swallow formulation within 120 seconds in USP dissolution apparatus 2 with 900 mL 0.0033 N hydrochloric acid at 30 rpm and 37°C. Even more preferably, at least 80% is dissolved in 120 seconds and even more preferably, at least 90% is dissolved in 120 seconds.

In one embodiment of a basic compound, the therapeutic compound is zolmitriptan. Preferably the carbonate is present in an amount between 1% and 50% by weight of the swallow formulation. More preferably the carbonate is present in an amount between 1% and 40%. More preferably the carbonate is present in an amount between 1mg and 450mg. Preferably the carbonate is sodium bicarbonate. Optionally the swallow formulation further comprises up to 50% by weight of a pharmaceutically acceptable acid.

In one embodiment the salt of a basic therapeutic compound is sildenafil citrate. Preferably the carbonate is present in an amount between 1% and 50% by weight of the swallow formulation. More preferably the carbonate is present in an amount between 1% and 40% by weight of the swallow formulation. More preferably the carbonate is present in an amount between 1mg and 450mg. Preferably the carbonate is a bicarbonate such as sodium bicarbonate or potassium bicarbonate or a mixture thereof. Optionally the swallow formulation further comprises up to 50% by weight of a pharmaceutically acceptable acid.

In a further embodiment the salt of a basic therapeutic compound is zolpidem tartrate. Preferably the carbonate is present in an amount between 1% and 50% by weight of the swallow formulation. More preferably the carbonate is present in an amount between 1% and 40% by weight of the swallow formulation. More preferably the carbonate is present in an amount between 1mg and 450mg. Preferably the carbonate is sodium bicarbonate. Optionally the swallow formulation may comprise up to 50% by weight of a pharmaceutically acceptable acid such as citric acid.

In yet a further embodiment the salt of a basic therapeutic compound is ondansetron hydrochloride. Preferably the carbonate is present in an amount between 1% and 50% by weight of the swallow formulation. More preferably the carbonate is present in an amount between 1mg and 450mg. More preferably the carbonate is present in an amount between 1% and 40% by weight of the swallow formulation. Preferably the carbonate is sodium bicarbonate. Optionally the swallow formulation may comprise up to 50% by weight of a pharmaceutically acceptable acid such as glycine.

In one embodiment the therapeutic compound is an amphoteric compound, cetirizine or a salt thereof. Preferably the carbonate is present in an amount between 1% and 50% by weight of the swallow formulation. More preferably the carbonate is present in an amount between 1% and 40% by weight of the swallow formulation. More preferably the carbonate is present in an amount between 1mg and 450mg. Preferably the carbonate is sodium bicarbonate. Optionally the swallow formulation may comprise up to 50% by weight of a pharmaceutically acceptable acid such as tartaric acid.

Optionally the swallow formulation may also comprise one or more pharmaceutically acceptable excipients or other components such as carriers, glidants, emulsifiers, diluents, binders, preservatives, wicking agents and/or disintegrants.

The swallow formulation may further contain flavouring agents, colouring agents and sweeteners.

In one embodiment the swallow formulation is co-administered with an aqueous fluid such as water. The co-administered fluid may be administered, before, after or with the swallow formulation.

Another aspect of the present invention is directed to a swallow formulation comprising a therapeutic compound that is a base, a salt of a base or an amphoteric compound or a salt of an amphoteric compound with an appropriate amount of one or more pH modulating agents wherein at least one pH modulating agent is a carbonate and which permits at least about 70% of the therapeutic compound to dissolve from the swallow formulation within 180 seconds in USP dissolution apparatus 2 with 900 mL 0.0033 N hydrochloric acid at 30 rpm and 37°C said dosage form further comprising one or more pharmaceutically acceptable carriers, diluents and/or excipients, wherein the swallow formulation is co-administered with fluid.

The swallow formulation may comprise one, two, three or more therapeutic agents.

Accordingly, in one preferred embodiment, the present invention provides a swallow formulation comprising two or more therapeutic compounds and one or more carbonates in an appropriate amount wherein at least one of the therapeutic compounds is a base, a salt of a base or an amphoteric compound or a salt of an amphoteric compound and at least 70% dissolves from the swallow formulation within 180 seconds in USP dissolution apparatus 2 with 900 mL 0.0033 N hydrochloric acid at 30 rpm and 37°C.

In another aspect of the present invention there is provided a dosage form comprising a swallow formulation comprising a therapeutic compound that is a base, a salt of a base or an amphoteric compound or a salt of an amphoteric compound and one or more pH modulating agents wherein at least one pH modulating agent is a carbonate in an amount from about 1% to about 50% by weight of the swallow formulation and wherein at least about 70% of the therapeutic compound is dissolved from the swallow formulation within 180 seconds in USP dissolution apparatus 2 with 900 mL 0.0033 N hydrochloric acid at 30 rpm and 37°C.

The dosage form may be a tablet, capsule, powder, cachet, paste, colloid, gel or melt. The dose form may optionally be in a chewable form.

The dosage form of the present invention may be coated, uncoated and/or layered tablet. Suitable coatings include water soluble polymer based coatings such as, povidone or hypromellose. Suitable coating polymers may also be a derivative of cellulose (cellulose acetophthalate, hypromellose phthalate) or a derivative of an acrylic polymer (methacrylate acid copolymer). Optionally, the dosage form may be coated with gelatin.

The dosage form may contain one or more further pharmaceutically active agents.

In one embodiment, the dosage form is a multi-phase release dosage form containing a further a therapeutic compound having a dissolution of less than 70% in 180 seconds in USP dissolution apparatus 2 with 900 mL 0.0033 N hydrochloric acid at 30 rpm and 37°C.

Swallow formulations of the present invention may be manufactured by admixing the ingredients simultaneously or sequentially and then converting into a dosage unit such as a tablet, capsule, wafer or the like.

Tablets of the present invention may be manufactured by direct compression or granulation and compression for example.

The present invention further contemplates a method for the amelioration or prevention of the symptoms associated with a disease or disorder, including pain, fever, discomfort, migraine, nausea, insomnia, sleep disorders, allergic rhinitis, atopy and erectile dysfunction in a subject, the method comprising administering to said subject a swallow formulation comprising a therapeutic compound that is a base, a salt of a base or an amphoteric compound or a salt of an amphoteric compound and one or more pH modulating agents wherein at least one of the pH modulating agents is a carbonate in an amount between 1% and 50% by weight of the swallow formulation, the therapeutic compound having enhanced dissolution from the swallow formulation, the administration being for a time and under conditions to prevent or ameliorate symptoms of the condition.

Conditions contemplated herein include any condition associated with a disease or disorder in need of treatment. Conditions include but are not limited to conditions associated with pain and/or fever, with the central nervous system, alimentary system, cardiovascular system, musculoskeletal system, respiratory system, allergy and immune system and genitourinary system, microbial infections, conditions requiring hormonal and steroidal treatment and conditions associated with the metabolism.

Another aspect of the present invention contemplates a method for management of a condition in a subject experiencing the condition or anticipating to experience the condition, said method comprising administering to said subject an oral delivery system comprising a therapeutic compound that is a base, a salt of a base, an amphoteric compound or a salt of an amphoteric compound to treat the condition and one or more pH modulating agents wherein at least one of the pH modulating agents is a carbonate in an amount between 1% and 50% by weight of the swallow formulation, the therapeutic compound having enhanced dissolution from the swallow formulation, the administration being for a time and under conditions to prevent or ameliorate symptoms of the condition.

These methods may also involve the oral dosage form having one or more pharmaceutically acceptable excipients.

As indicated above, the present invention extends to human, veterinary and animal husbandry applications.

The present invention is further described by the following non-limiting Examples.

Paracetamol is widely used as a marker for gastric emptying and absorption of high solubility and high permeability drugs. Along with scintigraphy, paracetamol is recognised as the gold standard for measuring rates of absorption. In a study on rapidly absorbed paracetamol tablets in 26 healthy fasted human subjects carried out by the applicant, *an in vitro-in vivo* correlation (IVIVC) was established between the AUC10 (R² = 0.951) and AUC20 (R² = 0.911) for paracetamol and the *in vitro* % drug dissolved in 180 seconds in USP apparatus 2 with 900 mL 0.0033 N hydrochloric acid at 30 rpm and 37°C. This quantity of dissolution medium contains 3 millimoles of hydrochloric acid.

Based on these correlations, a target in vitro dissolution rate of 70% dissolved in 180 seconds in USP apparatus 2 with 900 mL 0.0033 N hydrochloric acid at 30 rpm and 37°C was set as the target dissolution performance for formulations designed for rapid absorption.

In each of the Examples below, unless otherwise specified, all of the formulations are direct compression formulations prepared by blending the ingredients prior to compression. Formulation 1 is always the comparative example containing no carbonate. All other formulations contain carbonate in accordance with the present invention.

It will be apparent that the addition of a base such as a carbonate in accordance with the current invention substantially increases the dissolution of the basic active agents exemplified. For those compounds where the addition of carbonate alone does not provide adequate dissolution, surprisingly the addition of acid improved the dissolution significantly.

Example 1 is a base; Examples 2-4 are salts of bases and Example 5 is the salt of an amphoteric compound.

### EXAMPLE 1

### A Basic Compound

**Table 1 Zolmitriptan Formulations**

| **Formulation** | **1** | **2** |
|---|---|---|
| Sodium bicarbonate (mg) | 0 | 50 |
| Microcrystal cellulose (mg) | 110.3 | 97.1 |
| Sodium starch glycolate (mg) | 6 | 10 |
| Citric acid anhydrous(mg) | 0 | 38.4 |
| Zolmitriptan (mg) | 2.5 | 2.5 |
| Magnesium stearate (mg) | 1.2 | 2 |
| Carbonate (%) | 0 | 25 |
| Total (mg) | 120 | 200 |

**Table 2 Zolmitriptan Dissolution**

| **Formulation** | **% diss. at 90 sec** | **% diss. at 120 sec** | **% diss. at 180 sec** |
|---|---|---|---|
| **1** | 35.5 | 42.3 | 51.7 |
| **2** | 95.1 | 97.3 | 98.9 |

### EXAMPLE 2

### A Salt of a Basic Compound

**Table 3 Sildenafil Citrate Formulations**

| **Formulation** | **1** | **2** | **3** | **4*** |
|---|---|---|---|---|
| Sodium bicarbonate (mg) | 0 | 50 | 0 | 50 |
| Potassium bicarbonate (mg) | 0 | 0 | 50 | 0 |
| Microcrystalline cellulose (mg) | 370 | 320 | 280 | 255 |
| Croscarmellose sodium (mg) | 25 | 25 | 25 | 35 |
| Sildenafil citrate (mg) | 100 | 100 | 140 | 140 |
| Magnesium stearate (mg) | 5 | 5 | 5 | 5 |
| Povidone K-30 (mg) | 0 | 0 | 0 | 4.4 |
| Carbonate (%) | 0 | 10 | 10 | 10.2 |
| Total (mg) | 500 | 500 | 500 | 489.4 |

| | | | | |
|---|---|---|---|---|
| *Formulation 4 was prepared by wet granulation as outlined below | | | | |

**Table 4 Sildenafil Citrate Dissolution**

| **Formulation** | **% diss at 90 sec** | **% diss at 120 sec** | **% diss at 180 sec** |
|---|---|---|---|
| **1** | 13.2 | 15.2 | 18.2 |
| **2** | >99 | >99 | >99 |
| **3** | 89.5 | 93.5 | 97.3 |
| **4** | 94.4 | 97.5 | >99 |

**Table 5 Formulation 4 for a Sildenafil Citrate Granulation**

| **No** | **Ingredient** | **mg/tablet** |
|---|---|---|
| **Part 1** | | |
| 1 | Sildenafil citrate | 140 |
| 2 | Microcrystalline cellulose | 205 |
| 3 | Croscarmellose sodium | 20 |
| 4 | Povidone K-30 (PVP) | 4.4 |
| 5 | Water | ------ |
| **Part 2** | | |
| 5 | Sodium bicarbonate | 50 |
| 6 | Croscarmellose sodium | 15 |
| 7 | Microcrystalline cellulose | 50 |
| 8 | Magnesium stearate | 5 |

### Procedure

### Part 1

A. Prepare a 1.3%w/w solution of 4 in 5.
B. Blend 1,2 and 3.
C. Spray A onto B in a granulator or mixer to produce a granule suitable for compression.
D. Dry item C at 50°C to a moisture content ~3%.
E. Screen item D through a 850 micron sieve.

### Part 2

F. Screen ingredients 5-7 through a 250 micron sieve.
G. Blend Part 1 with F.
H. Screen ingredient 8 through a 250 micron sieve.
I. Blend G with H.
J. Compress.

### EXAMPLE 3

### A Salt of a Basic Compound

**Table 6 Zolpidem Tartrate Formulations**

| **Formulation** | **1** | **2** | **3** |
|---|---|---|---|
| Zolpidem tartrate (mg) | 10 | 10 | 10 |
| Sodium bicarbonate (mg) | 0 | 50 | 50 |
| Microcrystalline cellulose (mg) | 178 | 83 | 89.6 |
| Sodium starch glycolate (mg) | 10 | 10 | 10 |
| Tartaric acid 99% (mg) | 0 | 45 | 0 |
| Citric acid anhydrous (mg) | 0 | 0 | 38.4 |
| Magnesium stearate (mg) | 2 | 2 | 2 |
| Carbonate (%) | 0 | 25 | 25 |
| Total (mg) | 200 | 200 | 200 |

**Table 7 Zolpidem Tartrate Dissolution**

| **Formulation** | **% diss at 90 sec** | **% diss at 120 sec** | **% diss at 180 sec** |
|---|---|---|---|
| **1** | 49.4 | 55.8 | 62.3 20 |
| **2** | 89.4 | 91.8 | 92.6 |
| **3** | 94.4 | 95.9 | 96.1 |

### EXAMPLE 4

### A Salt of a Basic Compound

**Table 8 Ondansetron Hydrochloride Formulations**

| **Formulation** | **1** | **2** |
|---|---|---|
| Sodium bicarbonate (mg) | 0 | 20 |
| Microcrystalline cellulose (mg) | 180 | 140 |
| Crospovidone (mg) | 10 | 10 |
| Glycine (mg) | 0 | 18 |
| Ondansetron hydrochloride (mg) | 8 | 10 |
| Magnesium stearate (mg) | 2 | 2 |
| Carbonate (%) | 0 | 10 |
| Total (mg) | 200 | 200 |

**Table 9 Ondansetron Hydrochloride Dissolution**

| **Formulation** | **% diss at 90 sec** | **% diss at 120 sec** | **% diss at 180 sec** |
|---|---|---|---|
| **1** | 36.5 | 44.7 | 55.3 |
| **2** | 73.0 | 80.6 | 87.9 |

### EXAMPLE 5

### A Salt of an Amphoteric Compound

**Table 10 Cetirizine Dihydrochloride Formulations**

| **Formulation** | **1** | **2** | **3** |
|---|---|---|---|
| Cetirizine dihydrochloride (mg) | 10 | 10 | 10 |
| Sodium bicarbonate (mg) | 0 | 20 | 6 |
| Microcrystalline cellulose (mg) | 178 | 158 | 172 |
| Crospovidone (mg) | 10 | 10 | 10 |
| Magnesium stearate(mg) | 2 | 2 | 2 |
| Carbonate (%) | 0 | 10 | 3 |
| Total (mg) | 200 | 200 | 200 |

**Table 11 Cetirizine Dihydrochloride Dissolution**

| **Formulation** | **% diss at 90 sec** | **% diss at 120 sec** | **% diss at 180 sec** |
|---|---|---|---|
| **1** | 27.7 | 36.8 | 47.8 |
| **2** | 73.9 | 79 | 84.1 |
| **3** | 75.5 | 79.6 | 82.9 |

### BIBLIOGRAPHY

Grattan et al., Eur. J. Pharm. Biopharm 49(3):225-229, 2000
Jaronsinski P F and Hirschfeld S, N. Eng. J. Med. 325:1315-1316, 1991
Neuvonen, P. J. and ,Kivisto, K. T., Clin Pharmacokihet. 27 (2) 120-8, 1994

## Claims

1. A swallow formulation comprising:
a) a therapeutic compound that is a base, a salt of a base or an amphoteric compound or a salt of an amphoteric compound,
b) one or more pH modulating agents wherein at least one pH modulating agent is sodium bicarbonate or potassium bicarbonate in an amount that will neutralise 0.01 to 9.0 millimoles of hydrochloric acid and is present in an amount from about 1% to about 50% by weight of the swallow formulation, the amount of said bicarbonate being from 1 to 199 mg per tablet, and
c) a water uptake agent,
wherein at least about 70% of the therapeutic compound is dissolved from the swallow formulation within 180 seconds in United States Pharmacopeia (USP) dissolution apparatus 2 with 900 mL 0.0033 N hydrochloric acid at 30 rpm and 37°C.

2. Formulation according to claim 1 comprising two or more therapeutic compounds (a).

3. Formulation according to claim 1 or 2, wherein at least about 90% of the therapeutic compound is dissolved from the swallow formulation within 180 seconds in United States Pharmacopeia (USP) dissolution apparatus 2 with 900 mL 0.0033 N hydrochloric acid at 30 rpm and 37°C.

4. Formulation according to any one of claims 1 to 3, wherein the bicarbonate is sodium bicarbonate.

5. Formulation according to any of claims 1 to 4, comprising at least one pharmaceutically acceptable acid as a further pH modulating agent.

6. formulation according to claim 5, wherein the pharmaceutically acceptable acid is selected from citric acid, tartaric acid, succinic acid, ascorbic acid, malic acid, fumaric acid, metatartaric acid, adipic acid, sodium acid citrate, potassium acid citrate, glycine citrate, potassium acid tartrate, sodium acid tartrate, aspartic acid, glutamic acid, glycine, leucine, tyrosine, tryptophan, glycine fumarate, glycine hydrochloride, monophosphate, glycine and combinations thereof.

7. Formulation according to any of claims 1 to 6, wherein the water uptake agent (c) is selected from cross-linked polyvinylpyrrolidone (crospovidone), croscarmellose sodium, sodium starch glycolate, starch, starch derivatives, hydroxypropylcellulose, low substituted hydroxypropylcellulose, hydroxypropylmethylcellulose, alginic acid, sodium alginate, calcium sulphate, calcium carboxymethylcellulose, microcrystalline cellulose, powdered cellulose, colloidal silicon dioxide, docusate sodium, guar gum, magnesium aluminium silicate, methylcellulose, polacrilin potassium, silicified microcrystalline cellulose, magnesium oxide, tragacanth, mannitol, sorbitol, xylitol, sucrose, lactose, fructose, maltose, polyethylene glycol, aminoacids, cyclodextrin, urea and/or polyvinylpyrrolidone (povidone, PVP).

8. Formulation according to any of claims 1 to 7, wherein the therapeutic compound (a) is selected from the group consisting of analgesics such as opiates and opiate analogs, antipyretics, anti-migraine agents, sedatives, hypnotics, anti-anxiety agents, antipsychotic agents, antidepressants, anticonvulsants, antiemetics, antinauseants, expectorants, antitussives and decongestants, bronchodilators, antihistamines and anti-allergy agents, anti-diarrhoeals, antispasmodics and motility agents, hyperacidity, reflux and ulcer agents, antibiotics, antivirals and antifungals, detoxifying agents and agents used in drug dependence/ withdrawal and erectile dysfunction agents.

9. Formulation according to any of claims 1 to 8, wherein the therapeutic compound is selected from the group consisting of zolmitriptan, zolpidem, ondesetron, sildenafil, pseudoephedrine, dextromethorphan, chlorpheniramine, diphenhydramine and cetirizine, and pharmaceutically acceptable salts thereof.

10. Formulation according to any of claims 5 to 9, comprising a pharmaceutically acceptable acid in an amount up to 50% by weight of the swallow formulation.

11. Formulation according to any of claims 1 to 10 for its use in ameliorating symptoms associated with a disease or disorder.

## Patentansprüche

1. Schluckbare Formulierung, welche Folgendes umfasst:
a) eine therapeutische Verbindung, bei der es sich um eine Base, ein Salz einer Base oder eine amphotere Verbindung oder ein Salz einer amphoteren Verbindung handelt,
b) ein oder mehrere pH-modulierende Mittel, wobei es sich bei mindestens einem pH-modulierenden Mittel um Natriumbicarbonat oder Kaliumbicarbonat in einer Menge handelt, welche 0,01 bis 9,0 Millimol Salzsäure neutralisiert und in einer Menge von rund 1 bis rund 50 Gewichts-% der schluckbaren Formulierung vorliegt, wobei die Menge des besagten Bicarbonats 1 bis 199 mg pro Tablette beträgt, und
c) ein Wasseraufnahmemittel,
wobei in United States Pharmacopeia (USP) Auflösungsapparat 2 mit 900 ml 0,0033 N Salzsäure bei 30 U/min und 37 °C mindestens rund 70 % der therapeutischen Verbindung innerhalb von 180 Sekunden aus der schluckbaren Formulierung gelöst werden.

2. Formulierung nach Anspruch 1, welche zwei oder mehrere therapeutische Verbindungen (a) umfasst.

3. Formulierung nach Anspruch 1 oder 2, wobei in United States Pharmacopeia (USP) Auflösungsapparat 2 mit 900 ml 0,0033 N Salzsäure bei 30 U/min und 37 °C mindestens rund 90 % der therapeutischen Verbindung innerhalb von 180 Sekunden aus der schluckbaren Formulierung gelöst werden.

4. Formulierung nach einem der Ansprüche 1 bis 3, wobei es sich bei dem Bicarbonat um Natriumbicarbonat handelt.

5. Formulierung nach einem der Ansprüche 1 bis 4, welche mindestens eine pharmazeutisch verträgliche Säure als ein weiteres pH-modulierendes Mittel umfasst.

6. Formulierung nach Anspruch 5, wobei die pharmazeutisch verträgliche Säure ausgewählt ist aus Zitronensäure, Weinsäure, Bernsteinsäure, Ascorbinsäure, Apfelsäure, Fumarsäure, Metaweinsäure, Adipinsäure, natriumsaurem Citrat, kaliumsaurem Citrat, Glycincitrat, kaliumsaurem Tartrat, natriumsaurem Tartrat, Asparaginsäure, Glutaminsäure, Glycin, Leucin, Tyrosin, Tryptophan, Glycinfumarat, Glycinhydrochlorid, Monophosphat, Glycin und Kombinationen daraus.

7. Formulierung nach einem der Ansprüche 1 bis 6, wobei das Wasseraufnahmemittel (c) ausgewählt ist aus vernetztem Polyvinylpyrrolidon (Crospovidon), Croscarmellose-Natrium, Natriumstärkeglycolat, Stärke, Stärkederivaten, Hydroxypropylcellulose, niedrig substituierter Hydroxypropylcellulose, Hydroxypropylmethylcellulose, Alginsäure, Natriumalginat, Calciumsulfat, Calcium-Carboxymethylcellulose, mikrokristalliner Cellulose, pulverförmiger Cellulose, kolloidalem Siliciumdioxid, Docusat-Natrium, Guargummi, Magnesiumaluminiumsilicat, Methylcellulose, Polacrilin-Kalium, verkieselter mikrokristalliner Cellulose, Magnesiumoxid, Traganth, Mannitol, Sorbitol, Xylitol, Saccharose, Lactose, Fructose, Maltose, Polyethylenglycol, Aminosäuren, Cyclodextrin, Harnstoff und/oder Polyvinylpyrrolidon (Povidon, PVP).

8. Formulierung nach einem der Ansprüche 1 bis 7, wobei die therapeutische Verbindung (a) ausgewählt ist aus der Gruppe bestehend aus Analgetika wie Opiaten und Opiat-Analoga, Antipyretika, Migränemitteln, Sedativa, Hypnotika, Anxiolytika, Antipsychotika, Antidepressiva, Antikonvulsiva, Antiemetika, Mitteln gegen Übelkeit, Expektoranzien, Antitussiva und Dekongestiva, Bronchodilatatoren, Antihistaminen und Antiallergika, Antidiarrhoika, Spasmolytika und Motilika, Mitteln gegen Übersäuerung, Reflux und Magengeschwür, Antibiotika, Virostatika und Antimykotika, Entgiftungsmittel und Mitteln, die bei Drogenabhängigkeit/-entzug eingesetzt werden, und Mitteln gegen erektile Dysfunktion.

9. Formulierung nach einem der Ansprüche 1 bis 8, wobei die therapeutische Verbindung ausgewählt ist aus der Gruppe bestehend aus Zolmitriptan, Zolpidem, Ondansetron, Sildenafil, Pseudoephedrin, Dextromethorphan, Chlorpheniramin, Diphenhydramin und Cetirizin, und pharmazeutisch verträglichen Salzen davon.

10. Formulierung nach einem der Ansprüche 5 bis 9, welche eine pharmazeutisch verträgliche Säure in einer Menge von bis zu 50 Gewichts-% der schluckbaren Formulierung umfasst.

11. Formulierung nach einem der Ansprüche 1 bis 10 zu ihrer Verwendung in der Linderung von Symptomen, die mit einer Erkrankung oder Störung assoziiert sind.

## Revendications

1. Formulation à avaler comprenant :
a) un composé thérapeutique qui est une base, un sel d'une base ou un composé amphotère ou un sel d'un composé amphotère,
b) un ou plusieurs agent(s) de modulation de pH où au moins un agent de modulation de pH est du bicarbonate de sodium ou du bicarbonate de potassium en une quantité qui neutralisera 0,01 à 9,0 millimoles d'acide chlorhydrique et est présent en une quantité allant d'environ 1% à environ 50% en poids de la formulation à avaler, la quantité dudit bicarbonate étant comprise entre 1 et 199 mg par comprimé, et
c) un agent de reprise d'eau,
dans laquelle au moins environ 70% du composé thérapeutique sont dissous à partir de la formulation à avaler pendant 180 secondes dans un appareil de dissolution 2 de la Pharmacopée Américaine (USP) avec 900 ml d'acide chlorhydrique à 0,0033 N à 30 tr/min et 37°C.

2. Formulation selon la revendication 1 comprenant deux composés thérapeutiques (a) ou plus.

3. Formulation selon la revendication 1 ou 2, dans laquelle au moins environ 90% du composé thérapeutique sont dissous à partir de la formulation à avaler pendant 180 secondes dans un appareil de dissolution 2 de la Pharmacopée Américaine (USP) avec 900 ml d'acide chlorhydrique à 0,0033 N à 30 tr/min et 37°C.

4. Formulation selon l'une quelconque des revendications 1 à 3, dans laquelle le bicarbonate est du bicarbonate de sodium.

5. Formulation selon l'une des revendications 1 à 4, comprenant au moins un acide pharmaceutiquement acceptable comme un autre agent de modulation de pH.

6. Formulation selon la revendication 5, dans laquelle l'acide pharmaceutiquement acceptable est choisi parmi l'acide citrique, l'acide tartrique, l'acide succinique, l'acide ascorbique, l'acide malique, l'acide fumarique, l'acide métatartrique, l'acide adipique, le citrate disodique, le citrate acide de potassium, le citrate de glycine, le tartrate acide de potassium, le tartrate acide de sodium, l'acide aspartique, l'acide glutamique, la glycine, la leucine, la tyrosine, le tryptophane, le fumarate de glycine, le chlorhydrate de glycine, le monophosphate, la glycine et des combinaisons de ceux-ci.

7. Formulation selon l'une des revendications 1 à 6, dans laquelle l'agent de reprise d'eau (c) est choisi parmi la polyvinylpyrrolidone réticulée (crospovidone), le croscarmellose sodique, le glycolate d'amidon sodique, l'amidon, les dérivés d'amidon, l'hydroxypropylcellulose, l'hydroxypropylcellulose faiblement substituée, l'hydroxypropylméthylcellulose, l'acide alginique, l'alginate de sodium, le sulfate de calcium, la carboxyméthylcellulose de calcium, la cellulose microcristalline, la cellulose en poudre, la silice colloïdale, le docusate de sodium, la gomme de guar, le silicate d'aluminium et de magnésium, la méthylcellulose, la polacrilin-potassium, la cellulose microcristalline silicifiée, l'oxyde de magnésium, la gomme adragante, le mannitol, le sorbitol, le xylitol, le saccharose, le lactose, le fructose, le maltose, le polyéthylèneglycol, les acides aminés, la cyclodextrine, l'urée et/ou la polyvinylpyrrolidone (povidone, PVP).

8. Formulation selon l'une des revendications 1 à 7, dans laquelle le composé thérapeutique (a) est choisi dans le groupe constitué d'analgésiques tels que des opiacés et des analogues d'opiacés, des antipyrétiques, des agents antimigraineux, des sédatifs, des hypnotiques, des agents anxiolytiques, des agents antipsychotiques, des antidépresseurs, des anticonvulsivants, des antiémétiques, des antinauséeux, des expectorants, des antitussifs et des décongestionnants, des bronchodilatateurs, des antihistaminiques et des agents antiallergiques, des anti-diarrhéiques, des antispasmodiques et des agents de motilité, des agents contre l'hyperacidité, des agents anti-reflux et des agents antiulcéreux, des antibiotiques, des antiviraux et des antifongiques, des agents de détoxification et des agents utilisés dans la toxicomanie/le sevrage et des agents contre le dysfonctionnement érectile.

9. Formulation selon l'une des revendications 1 à 8, dans laquelle le composé thérapeutique est choisi dans le groupe constitué de zolmitriptan, de zolpidem, d'ondansétron, de sildénafil, de pseudoéphédrine, de dextrométhorphane, de chlorphéniramine, de diphénhydramine et de cétirizine et de sels pharmaceutiquement acceptables de ceux-ci.

10. Formulation selon l'une des revendications 5 à 9, comprenant un acide pharmaceutiquement acceptable en une quantité allant jusqu'à 50% en poids de la formulation à avaler.

11. Formulation selon l'une des revendications 1 à 10 pour son utilisation dans l'amélioration des symptômes associés à une maladie ou à un trouble.
